# EUROPEAN PATENT APPLICATION

(11) **EP 1 729 224 A2**
(43) Date of publication of application: **06.12.2006**
(21) Application number: 06252212.3
(22) Date of filing: 25.04.2006
(51) Int. Cl.: G06F 17/00

(54) **System for controlling the distribution of pharmaceuticals**

(30) Priority: 12.05.2005 GB 0509692
(71) Applicant: Jhetam, Imraan Feisal, Horseguards, Exeter, Devon EX4 4UY (GB)
(72) Inventor: Jhetam, Imraan Feisal, Horseguards, Exeter, Devon EX4 4UY (GB)
(74) Representative: Craske, Stephen Allan

(57) **Abstract**

A system for controlling the distribution of pharmaceuticals involves tracking the distribution of pharmaceutical products and managing pharmaceutical rebates via the internet. The system provides for the verification, payment and auditing of the rebates. Before the pharmacy can participate in the rebate scheme they must first undergo an initial registration process 110. Following registration, the pharmacy may purchase pharmaceutical products which are supplied from the manufacturer via a supply chain 120. The pharmaceuticals are supplied at a standard supply price, irrespective of the country in which the retailers are located. When the pharmaceuticals are supplied to an end user, e.g. a patient, the pharmacy enters a rebate claim 130, which is processed by an international clearing centre 140. Provided the processing has a successful outcome, the pharmacy receives a rebate payment 150, the size of which depends upon the country in which the pharmacist is located. This allows the dispensing of prescription pharmaceuticals at a discount to the patient and reduces the prevalence of parallel importation.

## Description

### TECHNICAL FIELD OF THE INVENTION

This invention relates to a system for controlling the distribution of pharmaceuticals.

### BACKGROUND

Parallel trade, or parallel importation, involves a product being traded across various borders under conditions which are not under the control of the product manufacturer. It is commonplace for manufacturers to sell their products in many different markets, and they often do so at different prices, depending on various factors pertaining to the market they are selling into. This has the effect of driving consumers and resellers to seek out markets where a particular product is cheapest, in order to save money or increase profit margins.

The pharmaceutical industry invests enormous amounts of money in research and development, and in order to recoup these costs they generally rely heavily on patents and other intellectual property laws to maintain price control in the various markets around the world. Parallel importation has the effect of reducing the ability of pharmaceutical companies to do this effectively, due to the wide variations in sale price which parallel importation can create within a particular market.

The pharmaceutical market is often subject to national government pricing regulations. Therefore, even if the individual companies themselves do not have different pricing structures for different markets, they may still find that their products are being sold for vastly different prices. This factor allows astute businesses to parallel import the pharmaceutical products from cheaper markets, such as Greece, into relatively expensive markets in places such as the United Kingdom and Denmark. The profit is made when suppliers and resellers buy the drugs in a country where the relevant govemment has negotiated the lowest prices from the pharmaceutical manufacturers and subsequently sell the products to consumers in the more expensive markets at a considerably higher price.

A major problem facing manufacturers is the fact that many parallel importers re-brand or re-package pharmaceutical products to bypass the import laws which are applicable to the market they are importing into. This may, for example, occur when packaging is changed or reduced in size, and when written material is translated into another language.

These activities have enormous impacts on pharmaceutical manufacturers, both in regard to profit margins, and also in relation to the lack of control which they have over their re-branded products. The existence of an uncontrolled supply route greatly increases the risk of counterfeit products entering the supply chain. Many feel that this type of parallel importation blatantly negates the value of trademarks held by the manufacturers, and many manufacturers have indeed challenged the repackaging of their products through the courts.

This frustration on the part of the pharmaceutical manufacturers has led to a variety of legal challenges. National and regional courts have been extremely busy considering cases involving parallel importation of pharmaceuticals, but to date the majority of rulings have broadly come down in favour of allowing parallel importation, albeit with some legally defined guidelines.

Previously, a process whereby the pharmacist would redeem a discount voucher by submitting the voucher to a separate control body to obtain reimbursement has been tried. This is inconvenient, and in many cases pharmacists have refused to accept the voucher system, with negative consequences for the patient. Pharmacists' refusal to supply a named product also tends to reflect unfavourably on the pharmaceutical company and its products. Furthermore, even though pharmaceutical companies using such a voucher system may obtain a broad indication of the demographic extent of use of the vouchers, and thus of their products, they cannot determine the extent to which the products are being prescribed by individual practitioners.

US 2004/205343 A1 to Forth et al. discloses a pharmaceutical tracking system having a server/database and code readers are provided at manufacturer and destination. The system server/database allocates a number of unique authentication codes to a manufacturer. An authentication code is applied to any level of product packaging, read by the manufacturer, and an activation request is communicated to the server/database for activation. Upon receipt of the product at the destination, only the authentication code activated by the manufacturer and indicated as such in the system sewer/database will verify that the product received is the one shipped. Code readers can be used at intermediate destinations to verify the authenticity of the product received and track the location of the product along the distribution chain.

US 6,859,780 B1 to Cunningham discloses a method of dispensing, tracking and managing pharmaceutical product samples in which prescribers and pharmacies are linked to a central computing station. Product trial media is magnetically encoded with information that identifies a particular pharmaceutical trial product. Participating prescribers activate the media via the central computing station before passing the activated media to patients who then present the media to participating pharmacies which validate the media via a link with the central computing station prior to dispensing the prescribed pharmaceutical. The central computing station includes a database that records data related to the use of the media so that all pharmaceutical trial products can be accounted for.

US 2003/074225 A1 to Borsand et al. discloses a system that reduces the number of processes needed to prescribe pharmaceuticals in accordance with the policies of a payor or PBM. The system can pre-certify prescriptions, check of for unfavourable pharmaceutical interactions and allergic reactions, prevent misuse of a prescription, monitor the filling and re-filling of a prescription, or cancel a prescription after it has been issued by a provider. The system centralizes the information storage into one or more locations that are accessible to all the appropriate entities.

US 2002/161607 A1 to Subich discloses a pharmaceutical sample tracking and control method. Manufacturers send samples bearing indicia to dispensing locations where a reader is used to read the information into a dispensing location database. As the samples are dispensed to patients, a reader is used to read the indicia into the database, and software tracks inventory levels by subtracting samples dispensed from the previous inventories. Manufacturers can access inventory levels at dispensing locations remotely, and practitioners can enter patient information, practitioner information, adverse reaction information and patient outcomes into the database.

US 4,949,256 to Humble discloses a coupon validation network for automatically processing product coupons which are presented for redemption by customers. The network includes a central control system having a database for redeemable coupons issued by manufacturers and a second database for coupons redeemed by retailers. Local control systems for operation by retailers store local database files, including a first file of redeemable coupons, a second file for all coupons redeemed by the retailer, and a third file for customer accounts of coupons presented by each customer. A coupon processing terminal for use by retailers adjusts the purchase price to reflect the number of coupons which have been redeemed and updates the second local database file. A second terminal for use by customers identifies valid coupons presented in advance for later redemption. Retailers can automatically process coupons presented for redemption, manufacturers can reimburse retainers for the value of the redeemed coupons, and customers can have permanent access to their coupons without carrying them.

US 5,581,064 to Riley et al. discloses an automated coupon processing system which inputs information from a manufacturer coupon, discerns a first identifying code from the coupon, correlates the first identifying code with one or more second identifying codes, and chooses a particular one of the second identifying codes, where the first identifying code and the chosen second identifying code uniquely identify the coupon. The system can prompt an operator to select one of the second identifying codes or to enter a coupon expiry date that corresponds to one of the second identifying codes. Coupons information can be entered using a bar code reader, an OCR scanner, or manual entry.

US Re. 37,166 E to Rando et al. discloses a point of sale (POS) bar code scanner having provision for reading redemption coupons in a manner secure from a human operator. Through decoding software, a comparison is made between information on a validation coupon and information on items presented for purchase, and a decision is made as to whether the redemption coupon is valid and redeemable in the transaction.

US 2001/0018664 A1 to Jacoves et al. discloses a system in which a rewards provider assembles reward program information and forwards it to a central office for implementation. The central office then transmits information to one or more stores, which provide rewards to customers based upon the purchase of discount triggering items. Upon meeting redemption requirements, the customer redeems the reward for gasoline. Redemption information received by the central office in the form of electronic files and redemption slips is then sent to a clearing house for processing. The clearing house invoices respective manufacturers for products sold and makes payments to the stores for the cost of the discount triggering items which were sold.

US 2001/0037236 A1 to Dixon, III. et al. discloses a coupon manager including a front end, back end and a central processor for processing and settlement of coupons. The coupons are received from customers at the front end which may include a cashier register. The cashier scans a bar code on the coupons and the scanned information is supplied to the back end. The received coupons are delivered to the back end for imaging and processing to determine whether the coupons are valid. The processed information is forwarded to the central processor which collates the information from a number of stores and reports the information to manufacturers for settlement. The specification also discloses a booklet having a listing of discounted items identifiable by a single bar code, which may be useable by itself or with the coupon manager.

US 4,971,362 to Lapsker discloses a prescription pad having a preprinted prescription leaf which bears a preprinted prescription for a distinct pharmaceutical product as well as a zone for entry of patient information and a zone for entry of the signature of the prescribing physician. A check leaf bears on one face a preprinted check in favour of a dispensing pharmacist, and has a value based on the value of the prescribed product and a dispensing fee. The check leaf also has an endorsing zone preprinted with a dispensing acknowledgement with a portion for entry of the endorsing signature of the dispensing pharmacist. The check leaf is preferably coded to identify the physician. The pad can be employed for the prescribing of free starter doses of a pharmaceutical product or to provide the patient with a discount. The pharmacist is reimbursed by depositing the endorsed preprinted check in his bank account. The control body on whose account the check is drawn is able to monitor the use of the preprinted prescriptions by physicians and provide the pharmaceutical company with valuable information.

US 2003/036957 to Nguyen discloses a method of avoiding the use of discount coupons. A number of products available for purchase from a number of vendors are advertised at a discount according to related discount coupons. Consumers purchase the advertised products without using the related discount coupons. Discount coupon data and proof of purchase data are sent over the internet to a clearing house. The vendors transmit purchase confirmation data to the clearing house, and the customers transmit requests for payment of the discounts to the discount payment clearing house. The clearing house then correlates the coupon data, proof of purchase data and purchase confirmation data and pays out cash discounts to the consumers.

It is an object of the present invention to provide a technology-based system for use in controlling the distribution of pharmaceuticals which complies with national price controls, is acceptable to retail outlets, and gives the end users access to a full range of prescription pharmaceuticals.

### SUMMARY OF THE INVENTION

The present invention provides a system for controlling the distribution of pharmaceuticals in which:
- pharmaceutical products are each marked with an identifying code prior to distribution to retail outlets via a supply chain, which code is distinguished from identifying codes applied to other pharmaceutical products of the same kind;
- the identifying codes are stored on a product database, accessible by a clearing centre, together with the destinations to which the products are sent;
- the clearing centre maintains a register of retail outlets located in regions where the products are sold at a reduced price, said register including the geographical locations of the registered retail outlets;
- the registered retail outlets read the identifying codes from the products before the products are issued to end users;
- the registered retail outlets send rebate claims including the identifying codes to the clearing centre;
   and
- the clearing centre checks the identifying codes in the received rebate claims against the product database and, provided the geographical location of the retail outlet which made the claim matches the product destination as stored in the product database, authorises payment of a monetary rebate to the retail outlet.

When viewed from another aspect, the invention also provides a system for controlling the distribution of pharmaceuticals which includes:
- maintaining a register of retail outlets located in regions where pharmaceutical products are to be sold at a reduced price, said register including the geographical locations of the registered retail outlets;
- accessing a product database containing identifying codes which are applied to pharmaceutical products prior to distribution to retail outlets via a supply chain, which codes are distinguished from identifying codes applied to other pharmaceutical products of the same kind, and the destinations to which the products are sent;
- receiving rebate claims from registered retail outlets, which rebate claims include identifying codes read from the products by the retail outlets before the products are issued to end users;
   and
- checking the identifying codes in the received rebate claims against the product database and, provided the geographical location of the retail outlet which made the claim matches the product destination as stored in the product database, authorising payment of a monetary rebate to the retail outlet.

The invention still further provides a system for controlling the distribution of pharmaceuticals which includes:
- obtaining entry on a register of retail outlets located in regions where pharmaceutical products are sold at a reduced price maintained by a clearing centre, which includes geographical locations of such retail outlets;
- reading an identifying code from a pharmaceutical product before the product is issued to an end user, which code is distinguished from identifying codes applied to other pharmaceutical products of the same kind, and which identifying codes are stored on a product database, accessible by the clearing centre, together with the destinations to which the products are sent;
- submitting rebate claims including the identifying codes to the clearing centre so that the clearing centre may check the identifying codes in the received rebate claims against the product database and, provided the geographical location of said entry matches the product destination as stored in the product database, authorise payment of a monetary rebate;
   and
- receiving payment of said monetary rebate.

The invention includes a system for controlling the distribution of pharmaceuticals which includes:
- marking pharmaceutical products with an identifying code prior to distribution to retail outlets via a supply chain, which code is distinguished from identifying codes applied to other pharmaceutical products of the same kind;
   and
- storing the identifying codes on a product database, accessible by a clearing centre, together with the destinations to which the products are sent.

The invention further includes a pharmaceutical product which is marked with an identifying code, which code is distinguished from identifying codes applied to other pharmaceutical products of the same kind, and said identifying codes are stored on a product database, accessible by a clearing centre, together with the destinations to which the products are sent.

In a preferred embodiment of the invention the retail outlets must undergo a registration process to be entered in the register of retail outlets. The registration process is preferably carried out using a computer at the retail outlet which communicates with a computer at the clearing centre via the intemet. The registration process may require various procedures, including:
- recording the name and address of the retail outlet in the register of retail outlets; and
- recording banking details of the retail outlet in the register of retail outlets.

Following registration, the retail outlet computers may communicate with the clearing centre computer via a secure password-protected internet connection. The identifying codes read by each retail outlet may also be communicated to the clearing centre using the password-protected internet connection.

The identifying codes marked on the pharmaceutical products are preferably unique to each product item constituting an individual packaged quantity of the product which is intended to be issued to an end user. The unique identifying codes are preferably applied in a machine-readable form, e.g. in the form of a barcode so that they can be read by each retail outlet using a barcode scanner. The identifying codes may be accompanied by a code which identifies the specific presentation and the particular manufacturer of the pharmaceutical product, i.e. a UPC (Universal Product Code). The acquired codes may be subjected to an integrity check before being processed by the clearing centre.

The identifying codes received by the clearing centre may be entered on a clearing database. The clearing centre may check the received identifying codes against the clearing database and reject any rebate claims made with identifying codes which have already been used.

The clearing centre may also keep a record of rebate claims to be audited. Rejected rebate claims may be automatically added to the record of rebate claims to be audited. A sample of rebate claims for which payment of a monetary rebate has been authorised may also be added to the record of rebate claims to be audited.

In the case of prescription pharmaceuticals, the identity of the prescribing doctor is preferably transmitted to the clearing centre in the rebate claim. The initials of a patient for which the prescription pharmaceuticals have been prescribed may also be transmitted to the clearing centre as well as the product identifying number. The clearing centre may authenticate rebate claims which are held in the record of rebate claims to be audited by contacting the medical practice of the prescribing doctor to check whether the pharmaceuticals have in fact been prescribed.

The rebate claims communicated to the clearing centre may include a request for payment of a monetary rebate value. For rebate claims for which payment of a monetary rebate has been authorised, the requests for payment of a monetary rebate value may be checked and, if necessary, adjusted to a value which is appropriate for the geographical area in which the rebate claim was made. The clearing centre may receive payments from the product manufacturer. A payment equal to the monetary rebate value may be made to the retail outlet concerned, preferably by electronic bank transfer direct to an appropriate bank account recorded in the register of retail outlets. Rebate claims for which payment of a monetary rebate have been authorised may be entered as such on the clearing database. Registered retail outlets may access the clearing database via a secure internet connection using a usemame and password to check the status of rebate claims which they have submitted.

### BRIEF DESCRIPTION OF THE DRAWINGS

The following description and the accompanying drawings referred to therein are included by way of non-limiting example in order to illustrate how the invention may be put into practice. In the drawings:
Figure 1 is a flow diagram of a pharmaceutical tracking and rebate system in accordance with the invention;
Figure 2 is a flow diagram showing details of the initial registration process which a retailer must undergo in order to use the system;
Figure 3 is a flow diagram view of the pharmaceutical supply chain from manufacturer to retailers;
Figure 4 is a flow diagram showing the acquisition of rebate claims from registered retailers;
Figure 5 is a flow diagram showing the processing of data in the course of a rebate claim;
Figure 6 is a flow diagram relating to the payment process;
Figure 7 is a flow diagram to show the audit process;
Figure 8 is general view of a pharmaceutical box showing the various barcodes and statutory information;
   and
Figure 9 is a flow diagram relating to the rebate notification system.

### DETAILED DESCRIPTION OF THE DRAWINGS

The preferred embodiment of the present invention and its advantages are best understood by referring to the accompanying drawings, in which like numerals refer to like elements.

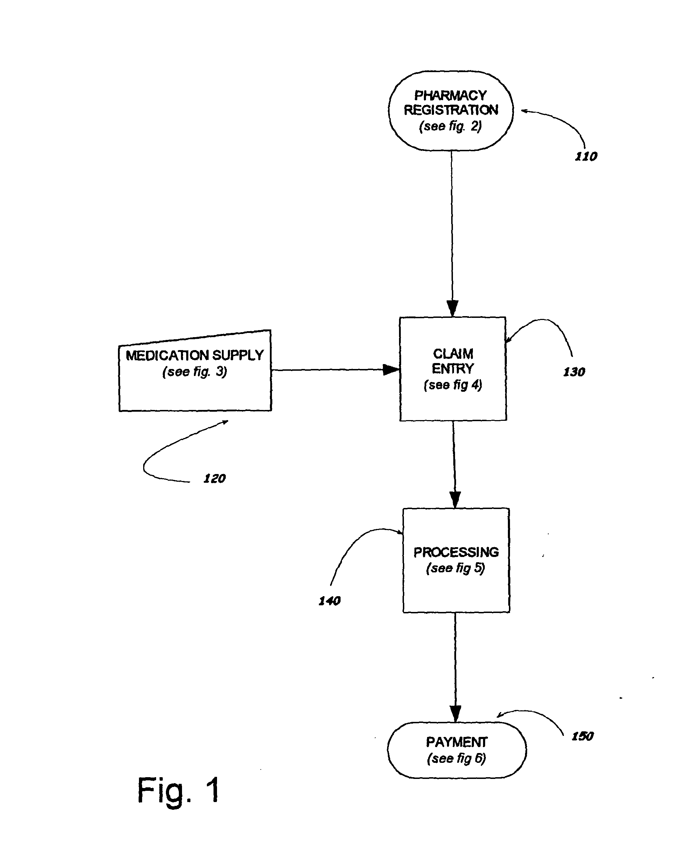

**Fig. 1** shows an overview of the preferred embodiment of the invention from the point of view of an individual retail outlet, represented in this instance by a pharmacy, which is located in a country where, due to national regulations or other factors, pharmaceutical products are sold to end users at a reduced price relative to the price at which end users in other countries receive the products. Before the pharmacy can participate in the rebate scheme they must first complete a registration process 110 in which the details of the pharmacy are entered on an electronic register. Following registration, the pharmacy may purchase pharmaceutical products which are received from the manufacturer via a supply chain 120 including one or more wholesalers. At the end of the supply chain the pharmaceuticals reach all retailers at a standard supply price for the products concerned, irrespective of the country in which the retailers are located. The supply price may apply to any group of countries, such as, for example, countries within the European Union or North America (USA and Canada). When the registered pharmacy supplies the pharmaceuticals to an end user, e.g. a patient, the pharmacy may enter a rebate claim 130, which is processed by an international clearing centre 140. Provided the processing has a successful outcome, the pharmacy receives a rebate payment 150, the size of which depends upon the country in which the pharmacy is located. The patient or other end user therefore receives the pharmaceuticals at a discounted price which has been agreed between the manufacturer and the relevant national body such as a national procurement authority or health authority. The price paid by the end user is equivalent to the standard supply price plus the markup (profit margin) applied by the pharmacy less the national rebate received by the pharmacy.

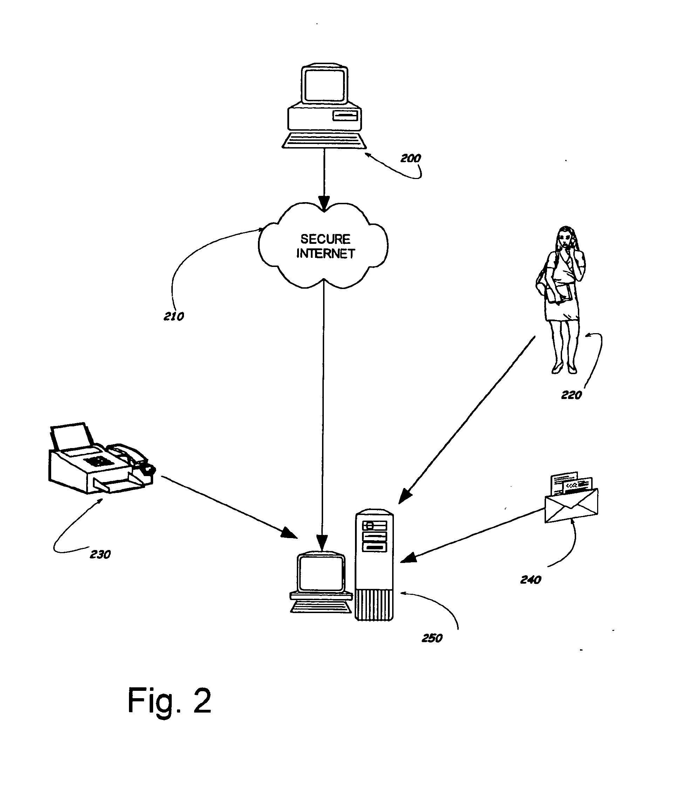

The registration process 110 is more fully shown in **Fig. 2.** A pharmacy applying for registration may submit their details using a pharmacy computer 200 which is connected to a computer at the international clearing centre 250 using a secure internet connection 210. The information required may include the name and national registration details of the pharmacy, ownership, banking information, postal address, telephone, fax and e-mail information. The information may also be submitted by the pharmacy using facsimile 230, post 240, or a clearing centre representative 220. Following initial checks to ensure that the pharmacy is a genuine retail outlet, the pharmacy is given a unique usemame and password for secure internet access to the clearing centre.

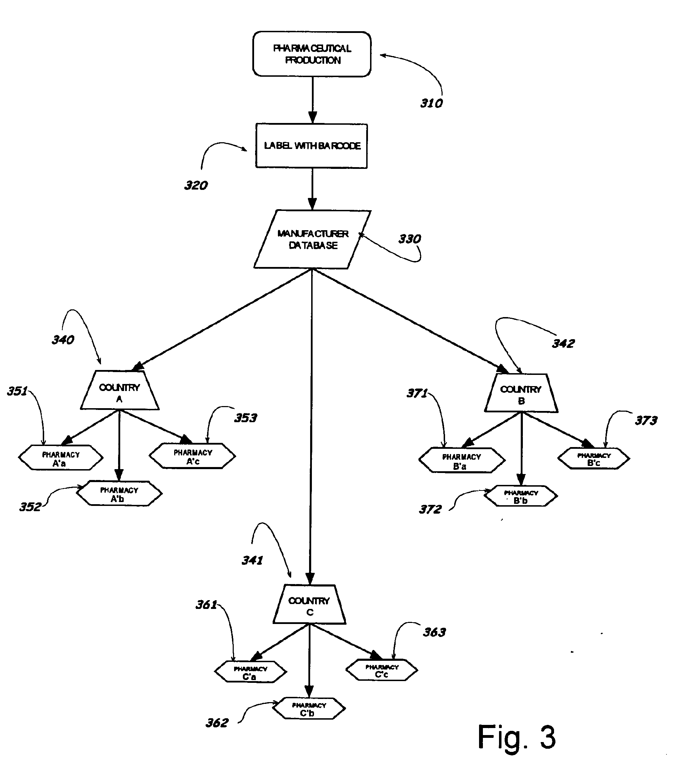

Referring now to **Fig. 3**, during pharmaceutical production 310, a unique identifying number is generated and assigned to each product item. A product item is the smallest quantity of the product which is packaged for supply to an end user. An example of a product item to which a unique identifying code would be applied is a box of pharmaceutical capsules or tablets which may be contained in blister packs inside the box, but not the blister packs themselves. Other examples include a tube of pharmaceutical product in the form of a cream or a similar product supplied in a screw-cap container. The unique identifying code is applied at 320 to the external packaging of the product item, which will usually be a box or other container. The code is printed or otherwise applied to the packaging in a permanent machine-readable form such as a barcode.

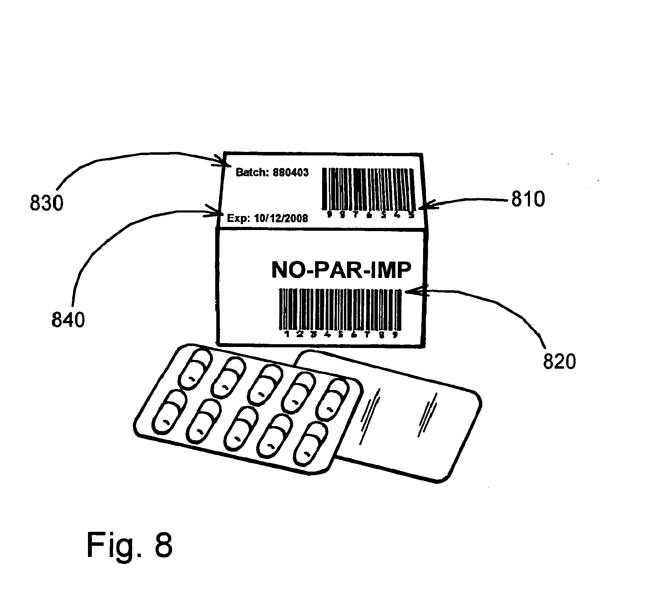

The unique identifying code may be applied in association with the usual UPC barcode which is used by retail outlets for product identification and pricing. (See **Fig. 8** below.) The UPC (Universal Product Code) contains various information about the nature of the product, such as whether the product is an ordinary item, a random weight item, a pharmaceutical product or a coupon. The code also identifies the manufacturer, and another part of the code is assigned by the manufacturer to identify the product according to weight, cost, size and other characteristics of the product. In the case of pharmaceuticals the UPC may also include their National Drug Control (NDC) number. It is, however, important to note that the UPC does not distinguish one product item from another, so that two boxes containing the same quantity of an identical product will both have the same UPC.

The identifying codes allocated to the product items are stored on a manufacturer's product database 330 for the product in question. The product database preferably allows the identifying codes to be linked with the UPC for the particular product. When the pharmaceutical product is distributed in country *A*, 340, for example, the country is also entered onto the database 330. Details of the individual pharmacies or other registered retailers to which the products are supplied such as pharmacy *A'a*, 351, pharmacy *A'b*, 352, or pharmacy *A'c*, 353, are preferably also entered on the product database if the information can be obtained from the supply chain. This enables a distinction to be made between a pharmaceutical product supplied for example to pharmacy *C'b*, 362 in country *C,* 341, or Pharmacy *B'a*, 361 in country *B,* 342.

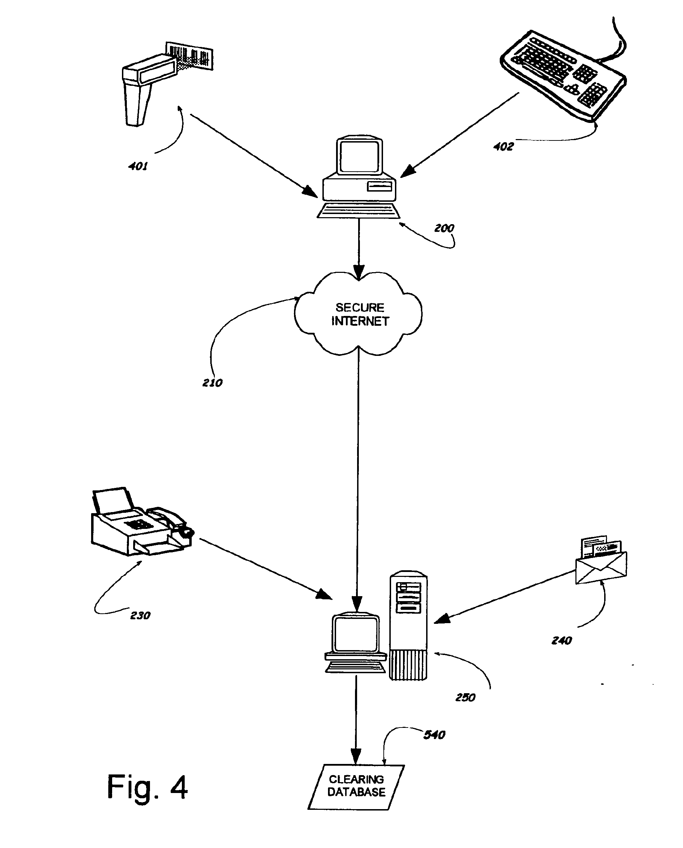

**Fig. 4** shows the acquisition of rebate claims by the clearing centre 250, which are entered by each pharmacy using the pharmacy computer 200 and a secure internet connection 210. When the products are prescribed by a medical practitioner for issue to a patient, the unique identifying numbers are obtained from the items to be dispensed by reading the barcodes using a handheld barcode scanner 401, which may be of known form. The UPC product barcode is also read at the same time. The barcodes can be read directly into the computer 200 from the scanner 401 or entered manually via a keyboard 402. As well as the barcode information, the rebate claim may also state the value of the rebate being claimed. In addition, the date on the prescription, the quantity of products being dispensed, the details of the prescribing doctor (e.g. name, address and telephone number) and information which is sufficient to identify the patient are also entered via the keyboard 402 and transmitted to the clearing centre 250. In order to comply with any relevant data protection regulations and ensure patient confidentiality only the initials and date of birth of the patient may be supplied to the clearing centre. When the information is received by the clearing centre 250 it is stored in a clearing database 540. The information could, altematively, be sent to the clearing centre by facsimile 230 or by post 240.

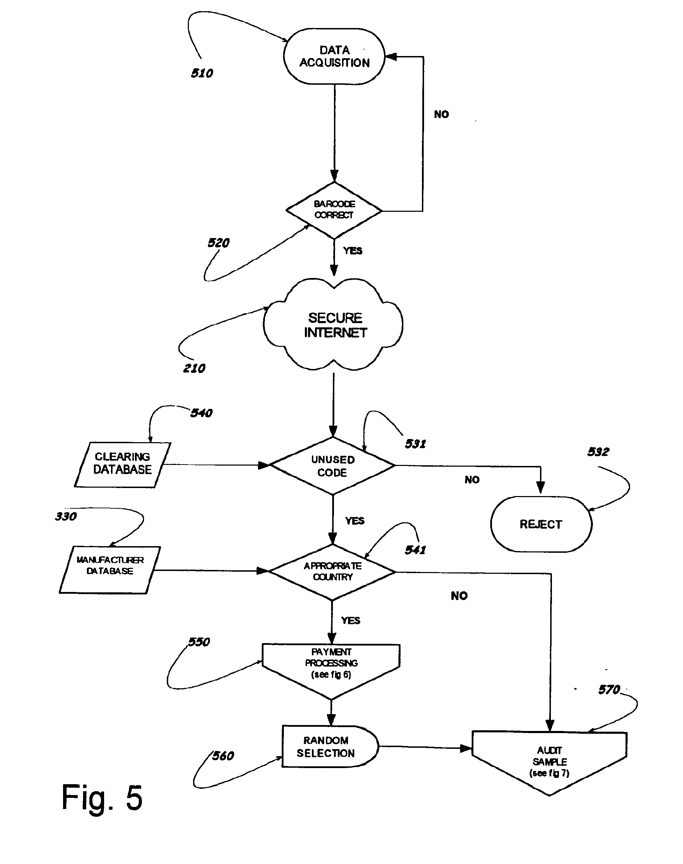

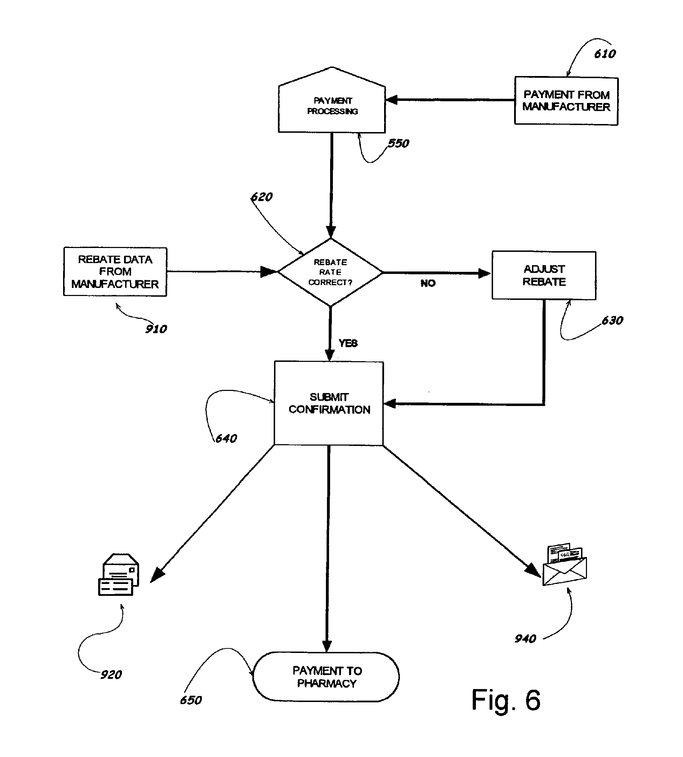

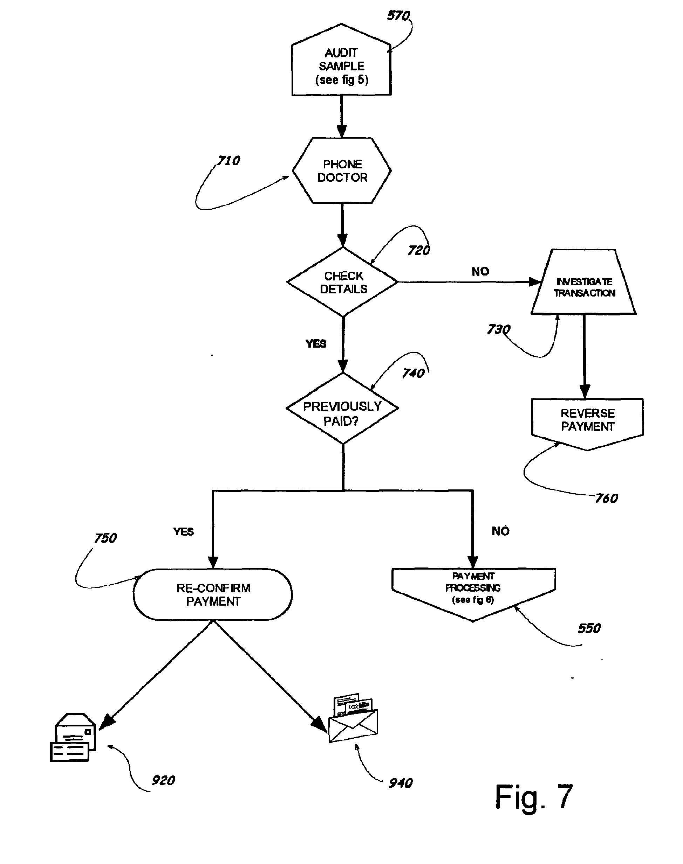

**Fig. 5** shows the data processing pathway 140 by which the rebate claim data is acquired and processed. The barcodes entered at 510 via the keyboard 402 or handheld scanner 401 are subjected to an initial integrity check 520, which may be carried out using an applet on a secure web page. If the codes are invalid, the user is required to re-acquire the barcode data. The acquired codes are transmitted to the clearing centre using the secure internet connection 210, as described. Upon receipt of a valid barcode a check 531 is made by the clearing centre computer against the clearing database 540 to ascertain if the unique identifying code relating to the pharmaceutical with that UPC is unused. If it has been used, the claim is rejected at 532.

The clearing centre computer then checks the manufacturers product database 330. If the supplied identifying code and UPC do not match the codes stored on the database the claim may be rejected. The database is also checked to ascertain if the product bearing the UPC and unique identifying number is being sold in the appropriate country to which it was distributed by the manufacturer. If the rebate is not being claimed in the country for which the products were intended, then the claim is automatically added to a record 570 of claims that need auditing.

If the pharmaceutical product bearing the unique identifying number and correct UPC is being sold in the appropriate country according to the product database 330, then the claim is sent for payment processing 550. A random sample from the claims sent for payment processing may be added to the record of claims that need auditing 570. Claims for a particular product may also be flagged for auditing and added to the record 570 if the number claims for that product made by a particular pharmacy show a significant increase.

Referring now to **Fig. 6**, claims passed for payment processing 550 are checked at 620 to see if the value of the rebate claimed is correct. If the rebate value is incorrect, it is adjusted at 630 to the value which is appropriate for the country concerned, as obtained from the manufacturer at 910.

When the rebate claimed is correct, and any necessary correction of the rebate value has been applied, the claim is approved and a confirmation advice 640 is submitted to the pharmacy by secure e-mail 920 and/or by post 940. The monetary value of the rebate is paid to the claiming pharmacy, 650, by bank transfer, cheque payment or other appropriate means.

The process for auditing the claims selected at 550 is shown in **Fig. 7.** The sample of claims to be audited 570 are each checked by means of a telephone call 710 made by a processing centre worker to the prescribing doctor's practice to check at 720 whether the patient with the submitted initials and date of birth did in fact receive a prescription with the supplied date for the pharmaceutical product in question. If the details are authenticated and the rebate payment has already been made, 740, then the payment is re-confirmed at 750, either by secure e-mail 920 and/or by post 940. If payment has not been made, the claim is submitted for processing 550 using the process described above in relation to **Fig. 6.**

If the details are not authenticated at 720 then the transaction is investigated at 730 and any payment is reversed at 760.

**Fig. 8** shows a typical product item in the form of a box of pharmaceuticals with the UPC marked on the box at 820. The batch number 830 is imprinted at production and packaging, as well as the expiry date 840. The unique identifying number 810, which is printed on the box in a machine-readable form during production and packaging, enables the individual box to be tracked in the distribution chain and permits authentication of the rebate, as described.

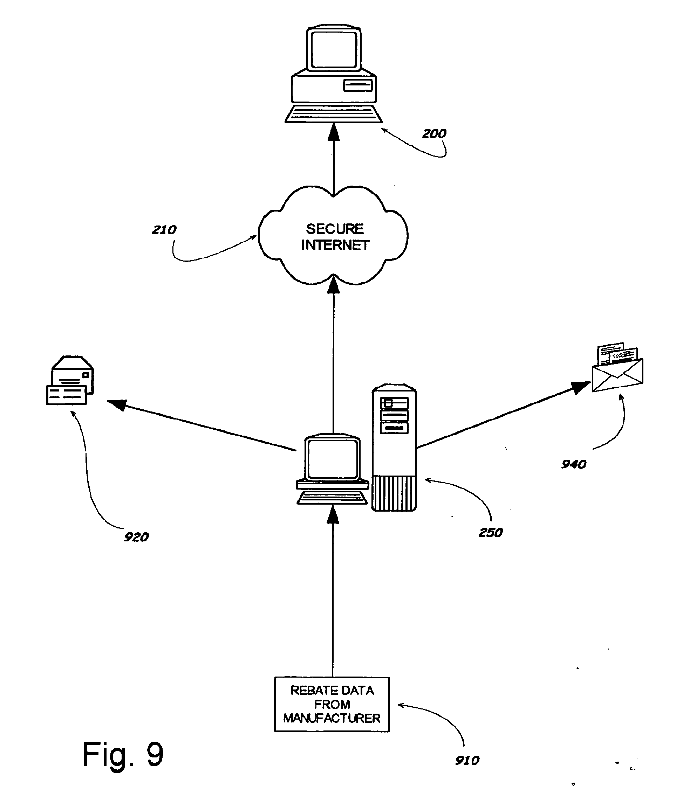

**Fig. 9** shows the process by which the clearing centre 250 handles an authenticated rebate claim. A monetary payment 910, appropriate to the product and geographical area of sale, is obtained from the manufacturer and the rebate is paid to the participating pharmacies by the clearing centre 250, preferably using electronic payment (wire transfer). The clearing centre notifies the pharmacy of the payment using secure e-mail 920 and/or by post 940.

The pharmacy can check details of all submitted, processed and paid claims by accessing the clearing centre database 540 using secure internet access with the supplied usemame and password. The pharmacy can also view the rebate values which can be claimed for any particular product.

The system of the present invention also facilitates product recall. The marking of the product packaging with a unique identifying code which is recorded in the manufacturers product database together and the existence of a tightly controlled supply chain enables individual products to be quickly traced and recalled at any point between the original manufacturer and the end user.

Although the above example describes a single international clearing centre 250 with a single computer it will be appreciated that the clearing centre may have multiple computers which are networked or otherwise linked together. Furthermore, the clearing centre need not necessarily be in one physical location and could, for example, be comprised of a number of regional processing centres working together.

The features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact method and operation illustrated and described, and accordingly, all suitable modifications and equivalents may be resorted to as falling within the scope of the invention.

It will be appreciated that the features disclosed herein may be present in any feasible combination. Whilst the above description lays emphasis on those areas which, in combination, are believed to be new, protection is claimed for any inventive combination of the features disclosed herein.

## Claims

1. A system for controlling the distribution of pharmaceuticals in which:
- pharmaceutical products are each marked with an identifying code (820) prior to distribution to retail outlets via a supply chain, which code is distinguished from identifying codes applied to other pharmaceutical products of the same kind;
- the identifying codes are stored on a product database (330), accessible by a clearing centre, together with the destinations to which the products are sent;
- the clearing centre maintains a computer register (250) of retail outlets located in regions where the products are sold at a reduced price, said computer register including the geographical locations of the registered retail outlets;
- retail outlets entered on the computer register (250) read the identifying codes (820) from the products before the products are issued to end users;
- the retail outlets send rebate claims including the identifying codes (820) to the clearing centre which are entered in a clearing database (540);
and
- the identifying codes (820) in the rebate claims entered in the clearing database (540) are electronically checked against the product database (330) and, provided the geographical location of the retail outlet which made the claim matches the product destination as stored in the product database, the clearing centre authorises payment of a monetary rebate to the retail outlet.

2. A system according to Claim 1 in which, to obtain entry in the register of retail outlets, the retail outlets undergo a registration process using a computer (200) which communicates with the clearing centre (250) via the internet and, following registration, the retail outlets communicate with the clearing centre via a secure password-protected internet connection (210).

3. A system according to Claim 1 or 2 in which the identifying codes (820) marked on the pharmaceutical products are unique to each product item which constitutes an individual packaged quantity of the product to be issued to an end user.

4. A system according to Claim 1, 2 or 3 in which the identifying codes (820) are applied to the products in a machine-readable form such as a barcode.

5. A system according to any preceding claim in which the identifying codes (820) applied to the products are accompanied by a code (810) which identifies the specific presentation and the particular manufacturer of the pharmaceutical product and said code (810) is read by each retail outlet and sent to the clearing centre in the rebate claim along with the identifying code.

6. A system according to any preceding claim in which the codes (820; 810) read by the retail outlets are subjected to an electronic integrity check before being passed to the clearing centre.

7. A system according to any preceding claim in which the clearing centre (250) electronically checks the received identifying codes (820) against the clearing database (540) and rejects any rebate claims made with identifying codes which have already been used.

8. A system according to any preceding claim in which the clearing centre (250) electronically selects and stores a record (570) of rebate claims to be audited.

9. A system according to any preceding claim in which, in the case of prescription pharmaceuticals, the identity of the prescribing doctor is transmitted to the clearing centre in the rebate claim.

10. A system according to any preceding claim in which the rebate claims communicated to the clearing centre include a request for payment of a monetary rebate value.
